# FASCICULE DE BREVET EUROPEEN

(11) **EP 1 129 689 B1**
(45) Date de publication et mention de la délivrance du brevet: **09.07.2008**
(21) Numéro de dépôt: 01400429.5
(22) Date de dépôt: 19.02.2001
(51) Int. Cl.: A61Q 5/10, A61K 8/42, A61K 8/40

(54) **Compositions pour la teinture d'oxydation des fibres kératiniques comprenant un N-(2-Hydroxybenzene)-carbamate ou un N-(2-Hydroxybenzene)-urée à titre de coupleur, et procédés de teinture**
Färbemittel für Keratinfasern enthaltend N-(2-Hydroxybenzol)carbamat- oder N-(2-Hydroxybenzol)harnstoff-Derivate als Kuppler und Färbungsverfahren
Keratinous fiber dyeing composition comprising N-(2-hydroxybenzene)-carbamate or N-(2-hydroxybenzene)urea derivatives as coupling agents and process for dyeing

(30) Priorité: 23.02.2000 FR 0002335
(43) Date de publication de la demande: 05.09.2001
(73) Titulaire: L'ORÉAL, 75008 Paris (FR)
(72) Inventeur: Saunier, Jean-Baptiste, 75014 Paris (FR); Vidal, Laurent, 75014 Paris (FR)
(74) Mandataire: Fevrier, Murielle Françoise E.

(56) Documents cités:
- WO-A-98/52519
- DD-A- 208 298
- FR-A- 2 233 984
- US-A- 3 674 414
- US-A- 5 334 225
- US-A- 5 676 706

## Description

L'invention a pour objet une composition pour la teinture d'oxydation des fibres kératiniques, et en particulier des fibres kératiniques humaines telles que les cheveux, comprenant au moins une base d'oxydation et, à titre de coupleur, au moins un N-(2-hydroxybenzène)-carbamate ou un N-(2-hydroxybenzène)-urée de formule (I), leur utilisation à titre de coupleur pour la teinture d'oxydation des fibres kératiniques, et les procédés de teinture d'oxydation les mettant en oeuvre.

Il est connu de teindre les fibres kératiniques et en particulier les cheveux humains avec des compositions tinctoriales contenant des précurseurs de colorant d'oxydation, en particulier des paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols, des naphtols non cationiques ou encore certains composés hétérocycliques tels que par exemples des coupleurs indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans les demandes de brevet WO 98/52519, JP2521636, FR-A-1 596 879 ou encore FR-A-2 233 984, des compositions de teinture d'oxydation contenant certains dérivés de N-(2-hydroxy-4-amino-phényl)-acétamide à titre de coupleurs, cependant de telles compositions ne sont pas toujours satisfaisantes, notamment du point de vue de la puissance des colorations obtenues.

US-A-5334225 décrit certains dérivés de N-(2-hydroxy-4-alkyl-phényl)-acétamide/carbamate/urée à titre de coupleurs.

Or, la demanderesse vient maintenant de découvrir, de façon totalement inattendue et surprenante, que certains composés de type N-(2-hydroxybenzène)-carbamates et N-(2-hydroxybenzène)-urées de formule (I) ci-après définie, non seulement conviennent pour une utilisation comme coupleur pour la teinture d'oxydation, mais en outre qu'ils permettent d'obtenir des compositions tinctoriales conduisant à des colorations puissantes, particulièrement chromatiques et brillantes, peu sélectives, dans une large palette de couleurs, et présentant d'excellentes propriétés de résistances aux différents traitements que peuvent subir les fibres kératiniques.

Ces découvertes sont à la base de la présente invention.

L'invention a donc pour premier objet une composition pour la teinture d'oxydation des fibres kératiniques, et en particulier des fibres kératiniques humaines telles que les cheveux, caractérisée par le fait qu'elle contient, dans un milieu approprié pour la teinture :
- au moins une base d'oxydation, et
- au moins un coupleur choisi parmi les composés de formule (I) suivante, et leurs sels d'addition avec un acide : 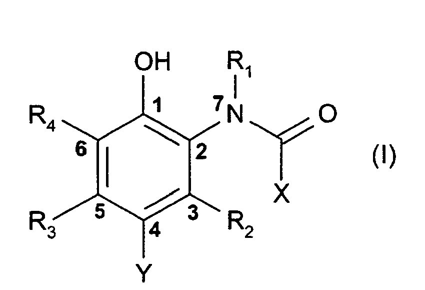
dans laquelle :
- R₁ représente un atome d'hydrogène ;
- X représente un radical méthoxy, éthoxy, propyloxy, isopropyloxy, butyloxy, isobutyloxy, 2-hydroxy-éthoxy, 2-méthoxy-éthoxy, allyloxy, amino, méthylamino, éthylamino, propylamino, isopropylamino, butylamino, isobutylamino, 2-hydroxy-éthylamino, 2-méthoxyéthylamino, 3-hydroxypropylamino, 2,3-dihydroxypropylamino, allylamino, diméthylamino, diéthylamino, di(2-hydroxy-éthyl)amino, pyrolidin-1-yle, 3-hydroxy-pyrolidin-1-yle, 3,4-dihydroxy-pyrolidin-1-yle ;
- R₂ représente un atome d'hydrogène ; ou un radical méthyle ;
- R₃ représente un radical amino, méthylamino, 2-hydroxyéthylamino, diethylamino méthane sulfonylamino, éthane sulfonylamino, ou diméthylamino sulfonylamino.
- R₄ représente un atome d'hydrogène, de chlore ou de fluor ; ou un groupement méthyle ;
- Y représente un atome d'hydrogène ou de chlore ; un groupement -OCH₂(CO)OCH₃ ou méthoxy.

Parmi les composés de formule (I) on peut citer :
- l'ester méthylique de l'acide (2-hydroxy-4-amino-phényl)-carbamique,
- l'ester éthylique de l'acide (2-hydroxy-4-amino-phényl)-carbamique,
- l'ester 2-hydroxy-éthylique de l'acide (2-hydroxy-4-amino-phényl)-carbamique,
- l'ester isopropylique de l'acide (2-hydroxy-4-amino-phényl)-carbamique,
- la 1-(2-hydroxy-4-amino-phényl)-urée,
- la 3-(2-hydroxy-4-amino-phényl)-1-méthyl-urée,
- la 3-(2-hydroxy-4-amino-phényl)-1-éthyl-urée,
- la 3-(2-hydroxy-4-amino-phényl)-1-(2-hydroxy-éthyl)-urée,
- la 3-(2-hydroxy-4-amino-phényl)-1-isopropyl-urée,
- la 3-(2-hydroxy-4-amino-phényl)-1,1-diméthyl-urée,
- l'ester méthylique de l'acide (2-hydroxy-4-méthylamino-phényl)-carbamique,
- l'ester éthylique de l'acide (2-hydroxy-4-méthylamino-phényl)-carbamique,
- l'ester 2-hydroxy-éthylique de l'acide (2-hydroxy-4-méthylamino-phényl)-carbamique,
- l'ester isopropylique de l'acide (2-hydroxy-4-méthylamino-phényl)-carbamique,
- la 1-(2-hydroxy-4-méthylamino-phényl)-urée,
- la 3-(2-hydroxy-4-méthylamino-phényl)-1-méthyl-urée,
- la 3-(2-hydroxy-4-méthylamino-phényl)-1-éthyl-urée,
- la 3-(2-hydroxy-4-méthylamino-phényl)-1-(2-hydroxy-éthyl)-urée,
- la 3-(2-hydroxy-4-méthylamino-phényl)-1 -isopropyl-urée,
- la 3-(2-hydroxy-4-méthylamino-phényl)-1,1-diméthyl-urée,
- l'ester méthylique de l'acide (2-hydroxy-4-(2-hydroxy-éthyl)amino-phényl)-carbamique,
- l'ester éthylique de l'acide (2-hydroxy-4-(2-hydroxy-éthyl)amino-phényl)-carbamique,
- l'ester 2-hydroxy-éthylique de l'acide (2-hydroxy-4-(2-hydroxy-éthyl)amino-phényl)-carbamique,
- l'ester isopropylique de l'acide (2-hydroxy-4-(2-hydroxy-éthyl)amino-phényl)-carbamique,
- la 1-(2-hydroxy-4-(2-hydroxy-éthyl)amino-phényl)-urée,
- la 3-(2-hydroxy-4-(2-hydroxy-éthyl)amino-phényl)-1-méthyl-urée,
- la 3-(2-hydroxy-4-(2-hydroxy-éthyl)amino-phényl)-1-éthyl-urée,
- la 3-(2-hydroxy-4-(2-hydroxy-éthyl)amino-phényl)-1-(2-hydroxy-éthyl)-urée,
- la 3-(2-hydroxy-4-(2-hydroxy-éthyl)amino-phényl)-1-isopropyl-urée,
- la 3-(2-hydroxy-4-(2-hydroxy-éthyl)amino-phényl)-1,1-diméthyl-urée,
- l'ester méthylique de l'acide (2-hydroxy-4-amino-5-chloro-phényl)-carbamique,
- l'ester éthylique de l'acide (2-hydroxy-4-amino-5-chloro-phényl)-carbamique,
- l'ester 2-hydroxy-éthylique de l'acide (2-hydroxy-4-amino-5-chloro-phényl)-carbamique,
- l'ester isopropylique de l'acide (2-hydroxy-4-amino-5-chloro-phényl)-carbamique,
- la 1-(2-hydroxy-4-amino-5-chloro-phényl)-urée,
- la 3-(2-hydroxy-4-amino-5-chloro-phényl)-1-méthyl-urée,
- la 3-(2-hydroxy-4-amino-5-chloro-phényl)-1-éthyl-urée,
- la 3-(2-hydroxy-4-amino-5-chloro-phényl)-1-(2-hydroxy-éthyl)-urée,
- la 3-(2-hydroxy-4-amino-5-chloro-phényl)-1-isopropyl-urée,
- la 3-(2-hydroxy-4-amino-5-chloro-phényl)-1,1-diméthyl-urée,
- l'ester méthylique de l'acide (2-hydroxy-4-méthylamino-5-chloro-phényl)-carbamique,
- l'ester éthylique de l'acide (2-hydroxy-4-méthylamino-5-chloro-phényl)-carbamique,
- l'ester 2-hydroxy-éthylique de l'acide (2-hydroxy-4-méthylamino-5-chloro-phényl)-carbamique,
- l'ester isopropylique de l'acide (2-hydroxy-4-méthylamino-5-chloro-phényl)-carbamique,
- la 1-(2-hydroxy-4-méthylamino-5-chloro-phényl)-urée,
- la 3-(2-hydroxy-4-méthylamino-5-chloro-phényl)-1-méthyl-urée,
- la 3-(2-hydroxy-4-méthylamino-5-chloro-phényl)-1-éthyl-urée,
- la 3-(2-hydroxy-4-méthylamino-5-chloro-phényl)-1-(2-hydroxy-éthyl)-urée,
- la 3-(2-hydroxy-4-méthylamino-5-chloro-phényl)-1-isopropyl-urée,
- la 3-(2-hydroxy-4-méthylamino-5-chloro-phényl)-1,1-diméthyl-urée,
- l'ester méthylique de l'acide (2-hydroxy-4-(2-hydroxy-éthyl)amino-5-chloro-phényl)-carbamique,
- l'ester éthylique de l'acide (2-hydroxy-4-(2-hydroxy-éthyl)amino-5-chloro-phényl)-carbamique,
- l'ester 2-hydroxy-éthylique de l'acide (2-hydroxy-4-(2-hydroxy-éthyl)amino-5-chloro-phényl)-carbamique,
- l'ester isopropylique de l'acide (2-hydroxy-4-(2-hydroxy-éthyl)amino-5-chloro-phényl)-carbamique,
- la 1-(2-hydroxy-4-(2-hydroxy-éthyl)amino-5-chloro-phényl)-urée,
- la 3-(2-hydroxy-4-(2-hydroxy-éthyl)amino-5-chloro-phényl)-1-méthyl-urée,
- la 3-(2-hydroxy-4-(2-hydroxy-éthyl)amino-5-chloro-phényl)-1-éthyl-urée,
- la 3-(2-hydroxy-4-(2-hydroxy-éthyl)amino-5-chloro-phényl)-1-(2-hydroxy-éthyl)-urée,
- la 3-(2-hydroxy-4-(2-hydroxy-éthyl)amino-5-chloro-phényl)-1-isopropyl-urée,
- la 3-(2-hydroxy-4-(2-hydroxy-éthyl)amino-5-chloro-phényl)-1,1-diméthyl-urée,
- l'ester méthylique de l'acide (2-hydroxy-4-amino-5-méthoxy-phényl)-carbamique,
- l'ester éthylique de l'acide (2-hydroxy-4-amino-5-méthoxy-phényl)-carbamique,
- l'ester 2-hydroxy-éthylique de l'acide (2-hydroxy-4-amino-5-méthoxy-phényl)-carbamique,
- l'ester isopropylique de l'acide (2-hydroxy-4-amino-5-méthoxy-phényl)-carbamique,
- la 3-(2-hydroxy-4-amino-5-méthoxy-phényl)-1-(2-hydroxy-éthyl)-urée,
- l'ester méthylique de l'acide (2-hydroxy-4-méthylamino-5-méthoxy-phényl)-carbamique,
- l'ester éthylique de l'acide (2-hydroxy-4-méthylamino-5-méthoxy-phényl)-carbamique,
- l'ester 2-hydroxy-éthylique de l'acide (2-hydroxy-4-méthylamino-5-méthoxy-phényl)-carbamique,
- l'ester isopropylique de l'acide (2-hydroxy-4-méthylamino-5-méthoxy-phényl)-carbamique,
- la 1-(2-hydroxy-4-méthylamino-5-méthoxy-phényl)-urée,
- la 3-(2-hydroxy-4-méthylamino-5-méthoxy-phényl)-1-méthyl-urée,
- la 3-(2-hydroxy-4-méthylamino-5-méthoxy-phényl)-1-éthyl-urée,
- la 3-(2-hydroxy-4-méthylamino-5-méthoxy-phényl)-1-(2-hydroxy-éthyl)-urée,
- la 3-(2-hydroxy-4-méthylamino-5-méthoxy-phényl)-1-isopropyl-urée,
- la 3-(2-hydroxy-4-méthylamino-5-méthoxy-phényl)-1,1-diméthyl-urée,
- l'ester méthylique de l'acide (2-hydroxy-4-(2-hydroxy-éthyl)amino-5-méthoxy-phényl)-carbamique,
- l'ester éthylique de l'acide (2-hydroxy-4-(2-hydroxy-éthyl)amino-5-méthoxy-phényl)-carbamique,
- l'ester 2-hydroxy-éthylique de l'acide (2-hydroxy-4-(2-hydroxy-éthyl)amino-5-méthoxy-phényl)-carbamique,
- l'ester isopropylique de l'acide (2-hydroxy-4-(2-hydroxy-éthyl)amino-5-éthoxy-phényl)-carbamique,
- la 1-(2-hydroxy-4-(2-hydroxy-éthyl)amino-5-méthoxy-phényl)-urée,
- la 3-(2-hydroxy-4-(2-hydroxy-éthyl)amino-5-méthoxy-phényl)-1-méthyl-urée,
- la 3-(2-hydroxy-4-(2-hydroxy-éthyl)amino-5-méthoxy-phényl)-1-éthyl-urée,
- la 3-(2-hydroxy-4-(2-hydroxy-éthyl)amino-5-méthoxy-phényl)-1-(2-hydroxy-éthyl)-urée,
- la 3-(2-hydroxy-4-(2-hydroxy-éthyl)amino-5-méthoxy-phényl)-1-isopropyl-urée,
- la 3-(2-hydroxy-4-(2-hydroxy-éthyl)amino-5-méthoxy-phényl)-1,1-diméthyl-urée,
et leurs sels d'addition avec un acide.

De façon encore plus préférentielle, les composés de formule (I) sont choisis parmi :
- l'ester méthylique de l'acide (2-hydroxy-4-amino-phényl)-carbamique,
- l'ester éthylique de l'acide (2-hydroxy-4-amino-phényl)-carbamique,
- l'ester 2-hydroxy-éthylique de l'acide (2-hydroxy-4-amino-phényl)-carbamique,
- l'ester isopropylique de l'acide (2-hydroxy-4-amino-phényl)-carbamique,
- la 1-(2-hydroxy-4-amino-phényl)-urée,
- la 3-(2-hydroxy-4-amino-phényl)-1-méthyl-urée,
- la 3-(2-hydroxy-4-amino-phényl)-1-éthyl-urée,
- la 3-(2-hydroxy-4-amino-phényl)-1-(2-hydroxy-éthyl)-urée,
- la 3-(2-hydroxy-4-amino-phényl)-1-isopropyl-urée,
- la 3-(2-hydroxy-4-amino-phényl)-1,1-diméthyl-urée,
- l'ester méthylique de l'acide (2-hydroxy-4-amino-5-chloro-phényl)-carbamique,
- l'ester éthylique de l'acide (2-hydroxy-4-amino-5-chloro-phényl)-carbamique,
- l'ester 2-hydroxy-éthylique de l'acide (2-hydroxy-4-amino-5-chloro-phényl)-carbamique,
- l'ester isopropylique de l'acide (2-hydroxy-4-amino-5-chloro-phényl)-carbamique,
- la 1-(2-hydroxy-4-amino-5-chloro-phényl)-urée,
- la 3-(2-hydroxy-4-amino-5-chloro-phényl)-1-méthyl-urée,
- la 3-(2-hydroxy-4-amino-5-chloro-phényl)-1-éthyl-urée,
- la 3-(2-hydroxy-4-amino-5-chloro-phényl)-1-(2-hydroxy-éthyl)-urée,
- la 3-(2-hydroxy-4-amino-5-chloro-phényl)-1-isopropyl-urée,
- la 3-(2-hydroxy-4-amino-5-chloro-phényl)-1,1-diméthyl-urée,
- l'ester méthylique de l'acide (2-hydroxy-4-amino-5-méthoxy-phényl)-carbamique,
- l'ester éthylique de l'acide (2-hydroxy-4-amino-5-méthoxy-phényl)-carbamique,
- l'ester 2-hydroxy-éthylique de l'acide (2-hydroxy-4-amino-5-méthoxy-phényl)-carbamique,
- l'ester isopropylique de l'acide (2-hydroxy-4-amino-5-méthoxy-phényl)-carbamique,
- la 3-(2-hydroxy-4-amino-5-méthoxy-phényl)-1-(2-hydroxy-éthyl)-urée, et leurs sels d'addition avec un acide.

Les composés de formule (I) conformes à l'invention sont des composés connus qui peuvent être préparées selon des procédés classiques bien connus de l'état de la technique et décrits par exemple dans les demandes de brevet et brevets JP 43008269, DE 2 156 480, US 4 310 527, JP 60108844, CS 235432, US 3 767 412, JP 02220047, JP 61035444 , JP 60237444, DE 3 524 519, EP 0 086 126, US 2 795 610 ou bien encore dans J. Heterocyclic Chem. 1983, 20, 1423, J. Mol. Struct. 1995, 344, 251.

Le ou les composés de formule (I) conformes à l'invention et/ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids.

La nature de la ou des bases d'oxydation pouvant être utilisées dans la composition tinctoriale conforme à l'invention n'est pas critique. Elles sont de préférence choisies parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.
Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2-méthyl pyrazolo-[1,5-a]-pyrimidine-3,7-diamine ; la 5-méthyl pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine, leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale conforme à l'invention peut également renfermer, en plus du ou des composés de formule (I) ci-dessus, un ou plusieurs coupleurs additionnels pouvant être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs additionnels représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (composés de formule (I), bases d'oxydation et coupleurs additionnels) sont notamment choisis parmi les chlorhydrates, les bromhydrates, les sulfates, les citrates, les succinates, les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 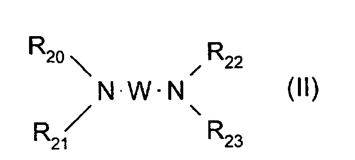 dans laquelle W est un reste propylène substitué ou non substitué par un groupement hydroxyle ou un radical alkyle en C₁-C₆ R₂₀, R₂₁, R₂₂ et R₂₃, identiques ou différents, représentent un atome d'hydrogène, un radical alkyle en C₁-C₆ ou hydroxyalkyle en C₁-C₆.

Les compositions de teinture d'oxydation conformes à l'invention peuvent également renfermer au moins un colorant direct, notamment pour modifier les nuances ou les enrichir en reflets.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet l'utilisation des composés de formule (I) telle que définie précédemment à titre de coupleur pour la teinture d'oxydation des fibres kératiniques et en particulier des fibres kératiniques humaines telles que les cheveux.

Un autre objet de l'invention est un procédé de teinture d'oxydation des fibres kératiniques et en particulier des fibres kératiniques humaines telles que les cheveux, mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides et les enzymes telles que les peroxydases, les laccases, les tyrosynases et les oxydoréductases parmi lesquelles on peut en particulier mentionner les pyranose oxydases, les glucose oxydases, les glycérol oxydases, les lactates oxydases, les pyruvate oxydases, et les uricases.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

L'invention a enfin pour objet un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES DE TEINTURE

### EXEMPLES 1 à 5 DE TEINTURE EN MILIEU ALCALIN

On a préparé les compositions tinctoriales suivantes (teneurs en grammes) :

| **EXEMPLE** | **1** | **2** | **3** | **4** | **4** |
|---|---|---|---|---|---|
| Paraphénylènediamine (base d'oxydation) | 0,72 | - | - | - | - |
| Para-amoniphénol (base d'oxydation) | - | 0,73 | - | - | - |
| 4,5-diamino 1-éthyl 3-méthyl pyrazole 2HCl (base d'oxydation) | - | - | 1,42 | - | - |
| Pyrazolo-[1,5-a]-pyrimidine-3,7-diamino, 2HCl (base d'oxydation) | - | - | - | 1,48 | - |
| N,N-bis hydroxyéthyl paraphébylènediamine, sulfate (base d'oxydation) | - | - | - | - | 1,96 |
| Ester éthylique de l'acide (2-hydroxy-4-amino-phényl)-carbamique (composé de formule (I)) | 1,31 | 1,31 | 1,31 | 1,31 | 1,31 |
| suport de teinture commun | (*) | (*) | (*) | (*) | (*) |
| Eau déminéralisée q.s.q. | 100 g | 100 g | 100 g | 100 g | 100 g |

| | | | | | |
|---|---|---|---|---|---|
| (*) Support de teinture commun: - Alcool oléique polyglycérolé à 2 moles de glycérol 4,0 g - Alcool oléique polyglycérolé à 24 moles de glycérol, à 78 % de matières actives (M.A.) 5,69 g M.A. - Acide oléique 3,0 g - amine oléique à 2 moles d'oxyde d'éthylène vendue sous la dénomination commerciale ETHOMEEN O12 par la société AKZO 7,0 g - Laurylamino succinamate de diéthylaminopropyle, sel de sodium à 55 % de M.A. 3,0 g M.A. - Alcool oléique 5,0 g - Diéthanolamide d'acide oléique 12,0 g - Propylèneglycol 3,5 g - Alcool éthylique 7,0 g - Dipropylèneglycol 0,5 g - Monométhyléther de propylèneglycol 9,0 g - Métabisulfite de sodium en solution aqueuse, à 35 % de M.A. 0,455 g M.A. - Acétate d'ammonium 0,8 g - antioxydant, séquestrant q.s. - Parfum, conservateur q.s. - Ammoniaque à 20 % de NH₃ 10,2 g | | | | | |

Au moment de l'emploi, on a mélangé chaque composition tinctoriale avec une quantité égale d'une composition oxydante constituée par une solution d'eau oxygénée à 20 volumes (6 % en poids) et présentant un pH d'environ 3.

Chaque mélange obtenu présentait un pH d'environ 9,5 et a été appliqué pendant 30 minutes sur des mèches de cheveux gris naturels à 90 % de blancs. Les mèches de cheveux ont ensuite été rincées, lavées avec un shampooing standard puis séchées.

Les mèches de cheveux ont été teintes dans les nuances figurant dans le tableau ci-dessous :

| **EXEMPLE** | **NUANCE SUR CHEVEUX NATURELS** |
|---|---|
| **1** | Acajou violacé |
| **2** | Blond cuivré doré |
| **3** | Blond irisé rouge |
| **4** | Blond cuivré doré |
| **5** | Bleu violacé |

### EXEMPLES 6 à 12 DE TEINTURE EN MILIEU ALCALIN

On a préparé les compositions tinctoriales suivantes (teneurs en grammes) :

| **EXEMPLE** | **6** | **7** | **8** | **9** | **10** | **11** | **12** |
|---|---|---|---|---|---|---|---|
| Paraphénylènediamine (base d'oxydation) | 0,72 | - | 0,72 | 0,72 | - | - | - |
| 4,5-diamino 1-éthyl 3-méthyl pyrazole 2HCl (base d'oxydation) | - | 1,42 | - | - | 1,42 | - | 1,42 |
| Pyrazolo-[1,5-a]-pyrimidine-3,7-diamino, 2HCl (base d'oxydation) | - | - | - | - | - | 1,48 | - |
| Ester méthylique de l'acide (2-hydroxy-4-amino-5-méthoxy-phényl)-carbamique (composé de formule (I)) | - | 1,66 | - | - | - | - | - |
| 3-(2-hydroxy-4-amino-phényl)-1-benzyl-urée (composé de formule (I)) | - | - | - | 1,71 | - | - | - |
| 3-(2-hydroxy-4-amino-phényl)-1-éthyl-urée (composé de formule (I)) | - | - | - | - | 1,30 | - | - |
| 3-(2-hydroxy-4-amino-phényl)-1-(2-hydroxy-éthyl)-urée (composé de formule (I)) | - | - | - | - | - | 1,41 | - |
| Chlorhydrate de la 3-(2-hydroxy-4-amino-phényl)-1,1-diméthyl-urée (composé de formule (I)) | - | - | - | - | - | - | 1,54 |
| Support de teinture commun | (*) | (*) | (*) | (*) | (*) | (*) | (*) |
| Eau déminéralisée q.s.p. | 100 | 100 | 100 | 100 | 100 | 100 | 100 |

| | | | | | | | |
|---|---|---|---|---|---|---|---|
| (*) Support de teinture commun : Il est identique à celui utilisé pour les exemples 1 à 5 ci-dessus. | | | | | | | |

Au moment de l'emploi, on a mélangé chaque composition tinctoriale avec une quantité égale d'une composition oxydante constituée par une solution d'eau oxygénée à 20 volumes (6 % en poids) et présentant un pH d'environ 3.

Chaque mélange obtenu présentait un pH d'environ 9,5 et a été appliqué pendant 30 minutes sur des mèches de cheveux gris naturels à 90 % de blancs. Les mèches de cheveux ont ensuite été rincées, lavées avec un shampooing standard puis séchées.

Les mèches de cheveux ont été teintes dans les nuances figurant dans le tableau ci-dessous :

| **EXEMPLE** | **NUANCE SUR CHEVEUX NATURELS** |
|---|---|
| **7** | Violine irisé |
| **9** | Blond cendré |
| **10** | Blond irisé rouge |
| **11** | Blond cuivré légèrement irisé |
| **12** | Blond irisé rouge |

## Revendications

1. Composition pour la teinture d'oxydation des fibres kératiniques, **caractérisée par le fait qu'**elle contient, dans un milieu approprié pour la teinture:
- au moins une base d'oxydation, et
- au moins un coupleur choisi parmi les composés de formule (1) suivante, et leurs sels d'addition avec un acide: 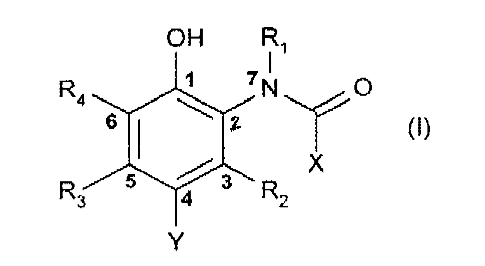
dans laquelle :
ii)
- R₁ représente un atome d'hydrogène ;
- X représente un radical méthoxy, éthoxy, propyloxy, isopropyloxy, butyloxy, isobutyloxy, 2-hydroxy-éthoxy, 2-méthoxy-éthoxy, allyloxy, amino, méthylamino, éthylamino, propylamino, isopropylamino, butylamino, isobutylamino, 2-hydroxy-ethylamino, 2-méthoxyéthylamino, 3-hydroxypropylamino, 2,3-dihydroxypropylamino, allylamino, diméthylamino, diéthylamino, di(2-hydroxy-éthyl)amino, pyrolidin-1-yle, 3-hydroxy-pyrolidin-1-yle, 3,4-dihydroxy-pyrolidin-1-yle ;
- R₂ représente un atome d'hydrogène ; ou un radical méthyle ;
- R₃ représente un radical amino, méthylamino, 2-hydroxy-éthylamino, diéthylamino, méthanesulfonylamino, éthanesulfonylamino, ou diméthylamino-sulfonylamino ;
- R₄ représente un atome d'hydrogène, de chlore, ou de fluor ; ou un groupement méthyle ;
- Y représente un atome d'hydrogène ou de chlore ; un groupement méthoxy, ou -OCH₂(CO)OCH₃.

2. Composition selon la revendication 1, **caractérisée par le fait que** les composés de formule (I) sont choisis parmi :
- l'ester méthylique de l'acide (2-hydroxy-4-amino-phényl)-carbamique,
- l'ester éthylique de l'acide (2-hydroxy-4-amino-phényl)-carbamique.,
- l'ester 2-hydroxy-éthylique de l'acide (2-hydroxy-4-amino-phényl)-carbamique,
- l'ester isopropylique de l'acide (2-hyd oxy-4-amino-phényl)-carbamique,
- la 1-(2-hydroxy-4-amino-phényl)-urée ,
- la 3-(2-hydroxy-4-amino-phényl)-1-mehyl-urée,
- la 3-(2-hydroxy-4-amino-phényl)-1-éthyl-urée,
- la 3-(2-hydroxy-4-amino-phényl)-1-(2. hydroxy-éthyl)-urée,
- la 3-(2-hydroxy-4-amino-phényl)-1-isopropyl-urée,
- la 3-(2-hydroxy-4-amino-phényl)-1,1-diméthyl-urée,
- la 3-(2-hydroxy-4-amino-phényl)-1,1-diéthyl-urée,
- la 3-(2-hydroxy-4-amino-phényl)-1,1-di(2-hydroxy-éthyl)-urée,
- le (4-amino-2-hydroxy-phényl)-amide de l'acide pyrrolidine-1-carboxylique,
- le (4-amino-2-hydroxy-phényl)-amide de l'acide 3-hydroxy-pyrrolidine-1-carboxylique,
- l'ester méthylique de l'acide (2-hydroxy-4-méthylamino-phényl)-carbamique,
- l'ester éthylique de l'acide (2-hydroxy-4-méthylamino-phényl)-carbamique,
- l'ester 2-hydroxy-éthylique de l'acide (2-hydroxy-4-méthylamino-phényl)-carbamique,
- l'ester isopropylique de l'acide (2-hydroxy-4-méthylamino-phényl)-carbamique,
- la 1-(2-hydroxy-4-méthylamino-phényl)-urée,
- la 3-(2-hydroxy-4-méthylamino-phényl)-1-méthyl-urée,
- la 3-(2-hydroxy-4-méthylamino-phényl)-1-éthyl-urée,
- la 3-(2-hydroxy-4-méthylamino-phényl)-1-(2-hydroxy-éthyl)-urée,
- la 3-(2-hydroxy-4-méthylamino-phényl)-1-isopropyl-urée,
- la 3-(2-hydroxy-4-méthylamino-phényl)-1,1-diméthyl-urée,
- la 3-(2-hydroxy-4-méthylamino-phényl)-1,1-diéthyl-urée,
- la 3-(2-hydroxy-4-méthylamino-phényl)-1,1-di(2-hydroxy-éthyl)-urée,
- l'ester méthylique de l'acide (2-hydroxy-4-(2-hydroxy-éthyl)amino-phényl)-carbamique,
- l'ester éthylique de l'acide (2-hydroxy-4-(2-hydroxy-éthyl)amino-phényl)-carbamique,
- l'ester 2-hydroxy-éthylique de l'acide (2-hydroxy-4-(2-hydroxy-éthyl)amino-phényl)-carbamique,
- l'ester isopropylique de l'acide (2-hydroxy-4-(2-hydroxy-éthyl)amino-phényl)-carbamique,
- la 1-(2-hydroxy-4-(2-hydroxy-éthyl)amino-phényl)-urée,
- la 3-(2-hydroxy-4-(2-hydroxy-éthyl)amino-phényl)-1-méthyl-urée,
- la 3-(2-hydroxy-4-(2-hydroxy-éthyl)amino-phényl)-1-éthyl-urée,
- la 3-(2-hydroxy-4-(2-hydroxy-éthyl)amino-phényl)-1-(2-hydroxy-éthyl)-urée,
- la 3-(2-hydroxy-4-(2-hydroxy-éthyl)amino-phényl)-1-isopropyl-urée,
- la 3-(2-hydroxy-4-(2-hydroxy-éthyl)amino-phényl)-1,1-diméthyl-urée,
- la 3-(2-hydroxy-4-(2-hydroxy-éthyl)amino-phenyl)-1,1-diéthyl-urée,
- la 3-(2-hydroxy-4-(2-hydroxy-éthyl)amino-phényl)-1,1-di(2-hydroxy-éthyl)-urée.
- l'ester méthylique de l'acide (2-hydroxy-4-amino-5-chloro-phényl)-carbamique,
- l'ester éthylique de l'acide (2-hydroxy-4-amino-5-chloro-phényl)-carbamique,
- l'ester 2-hydroxy-éthylique de l'acide (2-hydroxy-4-amino-5-chloro-phényl)-carbamique,
- l'ester isopropylique de l'acide (2-hydroxy-4-amino-5-chloro-phényl)-carbamique,
- la 1-(2-hydroxy-4-amino-5-chloro-phényl)-urée,
- la 3-(2-hydroxy-4-amino-5-chloro-phényl)-1-méthyl-urée,
- la 3-(2-hydroxy-4-amino-5-chloro-phényl)-1-éthyl-urée,
- la 3-(2-hydroxy-4-amino-5-chloro-phényl)-1-(2-hydroxy-éthyl)-urée,
- la 3-(2-hydroxy-4-amino-5-chloro-phényl)-1-isopropyl-urée,
- la 3-(2-hydroxy-4-amino-5-chloro-phényl)-1,1-diméthyl-urée,
- la 3-(2-hydroxy-4-amino-5-chloro-phényl)-1,1-diéthyl-urée,
- la 3-(2-hydroxy-4-amino-5-chloro-phényl)-1,1-di(2-hydroxy-éthyl)-urée,
- l'ester méthylique de l'acide (2-hydroxy-4-méthylamino-5-chloro-phényl)-carbamique,
- l'ester éthylique de l'acide (2-hydroxy-méthylamino-5-chloro-phényl)-carbamique,
- l'ester 2-hydroxy-éthylique de l'acide (2-hydroxy-4-méthylamino-5-chloro-phényl)-carbamique,
- l'ester isopropylique de l'acide (2-hydroxy-4-méthylamino-5-chloro-phényl)-carbamique,
- la 1-(2-hydroxy-4-méthylamino-5-chloro-phényl)-urée,
- la 3-(2-hydroxy-4-méthylamino-5-chloro-phényl)-1-méthyl-urée,
- la 3-(2-hydroxy-4-méthylamino-5-chloro-phényl)-1-éthyl-urée,
- la 3-(2-hydroxy-4-méthylamino-5-chloro-phényl)-1-(2-hydroxy-éthyl)-urée,
- la 3-(2-hydroxy-4-méthylamino-5-chloro-phényl)-1-isopropyl-urée,
- la 3-(2-hydroxy-4-méthylamino-5-chloro-phényl)-1,1-diméthyl-urée,
- la 3-(2-hydroxy-4-méthylamino-5-chloro-phényl)-1,1-diéthyl-urée,
- la 3-(2-hydroxy-4-méthylamino-5-chloro-phényl)-1,1-di(2-hydroxyéthyl)-urée.
- l'ester méthylique de l'acide (2-hydroxy-4-(2-hydroxy-éthyl)amino-5-chloro-phényl)-carbamique,
- l'ester éthylique de l'acide (2-hydroxy-4-(2-hydroxy-éthyl)amino-5-chloro-phényl)-carbamique,
- l'ester 2-hydroxy-éthylique de l'acide (2-hydroxy-4-(2-hydroxy-éthyl)amino-5-chloro-phényl)-carbamique,
- l'ester isopropylique de l'acide (2-hydroxy-4-(2-hydroxy-éthyl)amino-5-chloro-phényl)-carbamique.
- la 1-(2-hydroxy-4-(2-hydroxy-éthyl)amino-5-chloro-phényl)-urée,
- la 3-(2-hydroxy-4-(2-hydroxy-éthyl)amino-5-chloro-phényl)-1-méthyl-urée,
- la 3-(2-hydroxy-4-(2-hydroxy-éthyl)amino-5-chloro-phényl)-1 -éthyl-urée,
- la 3-(2-hydroxy-4-(2-hydroxy-éthyl)amino-5-chloro-phényl)-1-(2-hydroxy-éthyl)-urée,
- la 3-(2-hydroxy-4-(2-hydroxy-éthyl)amino-5-chloro-phényl)-1-isopropyl-urée,
- la 3-(2-hydroxy-4-(2-hydroxy-éthyl)amino-5-chloro-phényl)-1,1-diméthyl-urée,
- la 3-(2-hydroxy-4-(2-hydroxy-éthyl)amino-5-chloro-phényl)-1,1-diéthyl-urée,
- la 3-(2-hydroxy-4-(2-hydroxy-éthyl)amino-5-chloro-phényl)-1,1-di(2-hydroxy-éthyl)-urée,
- l'ester méthylique de l'acide (2-hydroxy-4-amino-5-méthoxy-phényl)-carbamique,
- l'ester éthylique de l'acide (2-hydroxy -4-amino-5-méthoxy-phényl)-carbamique,
- l'ester 2-hydroxy-éthylique de l'acide (2-hydroxy-4-amino-5-méthoxy-phényl)-carbamique,
- l'ester isopropylique de l'acide (2-hydroxy-4-amino-5-méthoxy-phényl)-carbamique,
- la 3-(2-hydroxy-4-amino-5-méthoxy-phényl)-1-(2-hydroxy-éthyl)-urée,
- la 3-(2-hydroxy-4-amino-5-méthoxy-phényl)-1,1-di(2-hydroxy-éthyl)-urée,
- l'ester méthylique de l'acide (2-hydroxy-4-méthylamino-5-méthoxy-phényl)-carbamique,
- l'ester éthylique de l'acide (2-hydroxy-4-méthylamino-5-méthoxy-phényl)-carbamique,
- l'ester 2-hydroxy-éthylique de l'acide (2-hydroxy-4-méthylamino-5-méthoxy-phényl)-carbamique,
- l'ester isopropylique de l'acide (2-hydroxy-4-méthylamino-5-méthoxy-phényl)-carbamique.
- la 1-(2-hydroxy-4-méthylamino-5-méthoxy-phényl)-urée,
- la 3-(2-hydroxy-4-méthylamino-5-méthoxy-phényl)-1-méthyl-urée,
- la 3-(2-hydroxy-4-méthylamino-5-méthoxy-phényl)-1-éthyl-urée,
- la 3-(2-hydroxy-4-méthylamino-5-méthoxy-phényl)-1-(2-hydroxy-éthyl)-urée,
- la 3-(2-hydroxy-4-méthylamino-5-méthoxy-phényl)-1-isopropyl-urée,
- la 3-(2-hydroxy-4-méthylamino-5-méthoxy-phényl)-1,1-diméthyl-urée,
- la 3-(2-hydroxy-4-méthylamino-5-méthoxy-phényl)-1,1-diéthyl-urée,
- la 3-(2-hydroxy-4-méthylamino-5-méthoaxy-phényl)-1,1-di(2-hydroxy-éthyl)-urée,
- l'ester méthylique de l'acide (2-hydroxy-4-(2-hydroxy-éthyl)amino-5-méthoxy-phényl)-carbamique,
- l'ester éthylique de l'acide (2-hydroxy-4-(2-hydroxy-éthyl)amino-5-méthoxy-phényl)-carbamique,
- l'ester 2-hydroxy-éthylique de l'acide (2-hydroxy-4-(2-hydroxy-éthyl)amino-5-méthoxy-phényl)-carbamique,
- l'ester isopropylique de l'acide (2-hydroxy-4-(2-hydroxy-éthyl)amino-5-méthoxy-phényl)-carbamique,
- la 1-(2-hydroxy-4-(2-hydroxy-éthyl)amino-5-méthoxy-phényl)-urée,
- la 3-(2-hydroxy-4-(2-hydroxy-éthyl)amino-5-méthoxy-phényl)-1-méthyl-urée,
- la 3-(2-hydroxy-4-(2-hydroxy-éthyl)amino-5-méthoxy-phényl)-1-éthyl-urée,
- la 3-(2-hydroxy-4-(2-hydroxy-éthyl)amino-5-méthoxy-phényl)-1-(2-hydroxy-éthyl)-urée,
- la 3-(2-hydroxy-4-(2-hydroxy-éthyl)amino-5-méthoxy-phényl)-1-isopropyl-urée,
- la 3-(2-hydroxy-4-(2-hydroxy-éthyl)amino-5,méthoxy-phényl)-1,1-diméthyl-urée,
- la 3-(2-hydroxy-4-(2-hydroxy-éthyl)amino-5-méthoxy-phényl)-1,1-diéthyl-urée,
- la 3-(2-hydroxy-4-(2-hydroxy-éthyl)amino-5-méthoxy-phényl)-1,1-di(2-hydroxy-éthyl)-urée.

3. Composition selon la revendication 1 ou 2, **caractérisée par le fait que** les composés de formule (I) sont choisis parmi :
- l'ester méthylique de l'acide (2-hydroxy-4-amino-phényl)-carbamique,
- l'ester éthylique de l'acide (2-hydroxy-4-amino-phényl)-carbamique,
- l'ester 2-hydroxy-éthylique de l'acide (2-hydroxy-4-amino-phényl)-carbamique,
- l'ester isopropylique de l'acide (2-hydroxy-4-amino-phényl)-carbamique,
- la 1-(2-hydroxy-4-amino-phényl)-urée,
- la 3-(2-hydroxy-4-amino-phényl)-1-méthyl-urée,
- la 3-(2-hydroxy-4-amino-phényl)-1-éthyl-urée,
- la 3-(2-hydroxy-4-amino-phényl)-1-(2-hydroxy-éthyl)-urée,
- la 3-(2-hydroxy-4-amino-phényl)-1-isopropyl-urée,
- la 3-(2-hydroxy-4-amino-phényl)-1,1-diméthyl-urée,
- l'ester méthylique de l'acide (2-hydroxy-4-amino-5-chloro-phényl)-carbamique,
- l'ester éthylique de l'acide (2-hydroxy-4-amino-5-chloro-phényl)-carbamique,
- l'ester 2-hydroxy-éthylique de l'acide (2-hydroxy-4-amino-5-chloro-phényl)-carbamique,
- l'ester isopropylique de l'acide (2-hydroxy-4-amino-5-chloro-phényl)-carbamique,
- la 1-(2-hydroxy-4-amino-5-chloro-phéhyl)-urée,
- la 3-(2-hydroxy-4-amino-5-chloro-phényl)-1-méthyl-urée,
- la 3-(2-hydroxy-4-amino-5-chloro-phényl)-1-éthyl-urée,
- la 3-(2-hydroxy-4-amino-5-chloro-phényl)-1-(2-hydroxy-éthyl)-urée,
- la 3-(2-hydroxy-4-amino-5-chloro-phényl)-1-isopropyl-urée,
- la 3-(2-hydroxy-4-amino-5-chloro-phényl)-1,1-diméthyl-urée,
- l'ester méthylique de l'acide (2-hydroxy-4-amino-5-méthoxy-phényl)-carbamique,
- l'ester éthylique de l'acide (2-hydroxy-4-amino-5-méthoxy-phényl)-carbamique,
- l'ester 2-hydroxy-éthylique de l'acide (2-hydroxy-4-amino-5-méthoxy-phényl)-carbamique,
- l'ester isopropylique de l'acide (2-hydroxy-4-amino-5-méthoxy-phényl)-carbamique,
- la 3-(2-hydroxy-4-amino-5-méthoxy-phényl)-1-(2-hydroxy-éthyl)-urée,
et leurs sels d'addition avec un acide.

4. Composition selon l'une quelconque des revendications précédentes, **caractérisée par le fait que** le ou les composés de formule (I) et/ou le ou leurs sels d'addition avec un acide représentent de préférence de 0,0005 à 12 % en poids du poids total de la composition tinctoriale.

5. Composition selon l'une quelconque des revendications précédentes, **caractérisée par le fait que** la ou les bases d'oxydation sont choisies parmi les paraphénylènediamines, les bis-phénylalkylènediamines, les para-aminophénols, les ortho-aminophénols et les bases hétérocycliques.

6. Composition selon la revendication 5, **caractérisée par le fait que** les paraphénylènediamines sont choisies parmi la paraphénylènediamine, la paratoluylènediamine, la 2-chloro paraphénylènediamine, la 2,3-diméthyl paraphénylènediamine, la 2,6-diméthg paraphénylènediamine, la 2,6-diéthyl paraphénylènediamine, la 2,5-diméthyl paraphénylénediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl) amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyèthyloxy paraphénylénediamine, la 2-β-acétylaminoéthyloxy paraphénylènediamine, la N-(β-méthoxyéthyl) paraphénylènediamine, et leurs sels d'addition avec un acide.

7. Composition selon la revendication 5, **caractérisée par le fait que** les bis-phénylalkylènediamines sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylénediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphènyl) éthylènediamine, le 1,8-bis-(2,5-diaminophénoxy)-3,5-dioxaoctane, et leurs sels d'addition avec un acide.

8. Composition selon la revendication 5, **caractérisée par le fait que** les para-aminophénols sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

9. selon la revendication 5, **caractérisée par le fait que** les ortho-aminophénols sont choisis parmi le 2-amino phénol, le 2-amino 5-méthyl phénol, le 2-amino 6-méthyl phénol, le 5-acétamido 2-amino phénol, et leurs sels d'addition avec un acide.

10. Composition selon la revendication 5, **caractérisée par le fait que** les bases hétérocycliques sont choisies parmi les dérivés pyridiniques, les dérivés pyrimidiniques et les dérivés pyrazoliques.

11. Composition selon l'une quelconque des revendications précédentes, **caractérisée par le fait que** la ou les bases d'oxydation représentent de 0,0005 à 12 % en poids du poids total de la composition tinctoriale.

12. Composition selon l'une quelconque des revendications précédentes, **caractérisée par le fait qu'**elle contient un ou plusieurs coupleurs additionnels choisis parmi les métaphénylènediamines, les méta-aminophénols, les métadiphénols et les coupleurs hétérocycliques et/ou au moins un colorant direct.

13. Composition selon la revendication 12, **caractérisée par le fait que** le ou les coupleurs additionnels représentent de 0,0001 à 10 % en poids du poids total de la composition tinctoriale.

14. Composition selon l'une quelconque des revendications précédentes, **caractérisée par le fait que** les sels d'addition avec un acide sont choisis parmi les chlorhydrates, les bromhydrates, les sulfates, les citrates, les succinates, les tartrates, les lactates et les acétates.

15. Utilisation des composés de formule (I) telle que définie à l'une quelconque des revendications 1 à 3, à titre de coupleur pour la teinture d'oxydation des fibres kératiniques et en particulier des fibres kératiniques humaines telles que les cheveux.

16. Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 1 à 3, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

17. Procédé selon la revendication 16, **caractérisé par le fait que** l'agent oxydant est choisi parmi le peroxyde d'hydrogène, le peroxyde d'urée, les bromates de métaux alcalins, les persels, et les enzymes.

18. Procédé selon la revendication 17 **caractérisé par le fait que** les enzymes sont choisies parmi les peroxydases, les laccases, les tyrosynases et les oxydoréductases.

19. Dispositif à plusieurs compartiments, ou "kit" de teinture à plusieurs compartiments, dont un premier compartiment renferme une composition tinctoriale telle que définie à l'une quelconque des revendications 1 à 14 et un second compartiment renferme une composition oxydante.

## Claims

1. Composition for the oxidation dyeing of keratinous fibres, **characterized in that** it comprises, in a medium appropriate for dyeing:
- at least one oxidation base, and
- at least one coupler chosen from compounds of the following formula (I) and their addition salts with an acid: 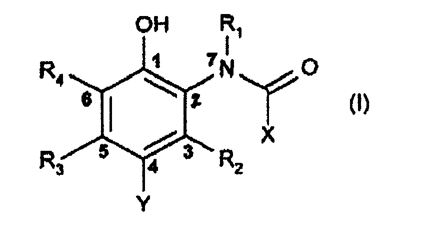
in which:
- R₁ represents a hydrogen atom;
- X represents a methoxy, ethoxy, propyloxy, isopropyloxy, butyloxy, isobutyloxy, 2-hydroxyethoxy, 2-methoxyethoxy, allyloxy, amino, methylamino, ethylamino, propylamino, isopropylamino, butylamino, isobutylamino, 2-hydroxyethylamino, 2-methoxyethylamino, 3-hydroxypropylamino, 2,3-dihydroxypropylamino, allylamino, dimethylamino, diethylamino, di(2-hydroxyethyl)amino, pyrrolidin-1-yl, 3-hydroxy-pyrrolidin-1-yl or 3,4-dihydroxypyrrolidin-1-yl radical;
- R₂ represents a hydrogen atom or a methyl radical;
- R₃ represents an amino, methylamino, 2-hydroxy-ethylamino, diethylamino, methanesulphonylamino, ethanesulphonylamino or dimethylamino-sulphonylamino radical;
- R₄ represents a hydrogen, chlorine or fluorine atom or a methyl group;
- Y represents a hydrogen or chlorine atom or a methoxy or -OCH₂(CO)OCH₃ group.

2. Composition according to Claim 1, **characterized in that** the compounds of formula (I) are chosen from:
- the methyl ester of 2-hydroxy-4-aminophenyl-carbamic acid,
- the ethyl ester of 2-hydroxy-4-aminophenylcarbamic acid,
- the 2-hydroxyethyl ester of 2-hydroxy-4-amino-phenylcarbamic acid,
- the isopropyl ester of 2-hydroxy-4-aminophenyl-carbamic acid,
- 1-(2-hydroxy-4-aminophenyl)urea,
- 3-(2-hydroxy-4-aminophenyl)-1-methylurea,
- 3-(2-hydroxy-4-aminophenyl)-1-ethylurea,
- 3-(2-hydroxy-4-aminophenyl)-1-(2-hydroxyethyl)urea,
- 3-(2-hydroxy-4-aminophenyl)-1-isopropylurea,
- 3-(2-hydroxy-4-aminophenyl)-1,1-dimethylurea,
- 3-(2-hydroxy-4-aminophenyl)-1,1-diethylurea,
- 3-(2-hydroxy-4-aminophenyl)-1,1-di(2-hydroxyethyl)urea,
- the 4-amino-2-hydroxyphenylamide of pyrrolidine-1-carboxylic acid,
- the 4-amino-2-hydroxyphenylamide of 3-hydroxy-pyrrolidine-1-carboxylic acid,
- the methyl ester of 2-hydroxy-4-(methylamino)phenylcarbamic acid,
- the ethyl ester of 2-hydroxy-4-(methylamino)phenylcarbamic acid,
- the 2-hydroxyethyl ester of 2-hydroxy-4-(methylamino)phenylcarbamic acid,
- the isopropyl ester of 2-hydroxy-4-(methylamino)phenylcarbamic acid,
- 1-(2-hydroxy-4-(methylamino)phenyl)urea,
- 3-(2-hydroxy-4-(methylamino)phenyl)-1-methylurea,
- 3-(2-hydroxy-4-(methylamino)phenyl)-1-ethylurea,
- 3-(2-hydroxy-4-(methylamino)phenyl)-1-(2-hydroxyethyl)urea,
- 3-(2-hydroxy-4-(methylamino)phenyl)-1-isopropyl-urea,
- 3-(2-hydroxy-4-(methylamino)phenyl)-1,1-dimethyl-urea,
- 3-(2-hydroxy-4-(methylamino)phenyl)-1,1-diethyl-urea,
- 3-(2-hydroxy-4-(methylamino)phenyl)-1,1-di(2-hydroxyethyl)urea,
- the methyl ester of 2-hydroxy-4-((2-hydroxyethyl)amino)phenylcarbamic acid,
- the ethyl ester of 2-hydroxy-4-((2-hydroxyethyl)amino)phenylcarbamic acid,
- the 2-hydroxyethyl ester of 2-hydroxy-4-((2-hydroxyethyl)amino)phenylcarbamic acid,
- the isopropyl ester of 2-hydroxy-4-((2-hydroxyethyl)amino)phenylcarbamic acid,
- 1-(2-hydroxy-4-((2-hydroxyethyl)amino)phenyl)urea,
- 3-(2-hydroxy-4-((2-hydroxyethyl)amino)phenyl)-1-methylurea,
- 3-(2-hydroxy-4-((2-hydroxyethyl)amino)phenyl)-1-ethylurea,
- 3-(2-hydroxy-4-((2-hydroxyethyl)amino)phenyl)-1-(2-hydroxyethyl)urea,
- 3-(2-hydroxy-4-((2-hydroxyethyl)amino)phenyl)-1-isopropylurea,
- 3-(2-hydroxy-4-((2-hydroxyethyl)amino)phenyl)-1,1-dimethylurea,
- 3-(2-hydroxy-4-((2-hydroxyethyl)amino)phenyl)-1,1-diethylurea,
- 3-(2-hydroxy-4-((2-hydroxyethyl)amino)phenyl)-1,1-di(2-hydroxyethyl)urea,
- the methyl ester of 2-hydroxy-4-amino-5-chloro-phenylcarbamic acid,
- the ethyl ester of 2-hydroxy-4-amino-5-chloro-phenylcarbamic acid,
- the 2-hydroxyethyl ester of 2-hydroxy-4-amino-5-chlorophenylcarbamic acid,
- the isopropyl ester of 2-hydroxy-4-amino-5-chloro-phenylcarbamic acid,
- 1-(2-hydroxy-4-amino-5-chlorophenyl)urea,
- 3-(2-hydroxy-4-amino-5-chlorophenyl)-1-methylurea,
- 3-(2-hydroxy-4-amino-5-chlorophenyl)-1-ethylurea,
- 3-(2-hydroxy-4-amino-5-chlorophenyl)-1-(2-hydroxyethyl)urea,
- 3-(2-hydroxy-4-amino-5-chlorophenyl)-1-isopropyl-urea,
- 3-(2-hydroxy-4-amino-5-chlorophenyl)-1,1-dimethyl-urea,
- 3-(2-hydroxy-4-amino-5-chlorophenyl)-1,1-diethyl-urea,
- 3-(2-hydroxy-4-amino-5-chlorophenyl)-1,1-di(2-hydroxyethyl)urea,
- the methyl ester of 2-hydroxy-4-methylamino-5-chlorophenylcarbamic acid,
- the ethyl ester of 2-hydroxy-4-methylamino-5-chlorophenylcarbamic acid,
- the 2-hydroxyethyl ester of 2-hydroxy-4-methylamino-5-chlorophenylcarbamic acid,
- the isopropyl ester of 2-hydroxy-4-methylamino-5-chlorophenylcarbamic acid,
- 1-(2-hydroxy-4-methylamino-5-chlorophenyl)urea,
- 3-(2-hydroxy-4-methylamino-5-chlorophenyl)-1-methylurea,
- 3-(2-hydroxy-4-methylamino-5-chlorophenyl)-1-ethylurea,
- 3-(2-hydroxy-4-methylamino-5-chlorophenyl)-1-(2-hydroxyethyl)urea,
- 3-(2-hydroxy-4-methylamino-5-chlorophenyl)-1-isopropylurea,
- 3-(2-hydroxy-4-methylamino-5-chlorophenyl)-1,1-dimethylurea,
- 3-(2-hydroxy-4-methylamino-5-chlorophenyl)-1,1-diethylurea,
- 3-(2-hydroxy-4-methylamino-5-chlorophenyl)-1,1-di(2-hydroxyethyl)urea,
- the methyl ester of 2-hydroxy-4-((2-hydroxyethyl)amino)-5-chlorophenylcarbamic acid,
- the ethyl ester of 2-hydroxy-4-((2-hydroxyethyl)amino)-5-chlorophenylcarbamic acid,
- the 2-hydroxyethyl ester of 2-hydroxy-4-((2-hydroxyethyl)amino)-5-chlorophenylcarbamic acid,
- the isopropyl ester of 2-hydroxy-4-((2-hydroxyethyl)amino)-5-chlorophenylcarbamic acid,
- 1-(2-hydroxy-4-((2-hydroxyethyl)amino)-5-chlorophenyl)urea,
- 3-(2-hydroxy-4-((2-hydroxyethyl)amino)-5-chlorophenyl)-1-methylurea,
- 3-(2-hydroxy-4-((2-hydroxyethyl)amino)-5-chlorophenyl)-1-ethylurea,
- 3-(2-hydroxy-4-((2-hydroxyethyl)amino)-5-chlorophenyl)-1-(2-hydroxyethyl)urea,
- 3-(2-hydroxy-4-((2-hydroxyethyl)amino)-5-chlorophenyl)-1-isopropylurea,
- 3-(2-hydroxy-4-((2-hydroxyethyl)amino)-5-chlorophenyl)-1,1-dimethylurea,
- 3-(2-hydroxy-4-((2-hydroxyethyl)amino)-5-chlorophenyl)-1,1-diethylurea,
- 3-(2-hydroxy-4-((2-hydroxyethyl)amino)-5-chlorophenyl)-1,1-di(2-hydroxyethyl)urea,
- the methyl ester of 2-hydroxy-4-amino-5-methoxy-phenylcarbamic acid,
- the ethyl ester of 2-hydroxy-4-amino-5-methoxy-phenylcarbamic acid,
- the 2-hydroxyethyl ester of 2-hydroxy-4-amino-5-methoxyphenylcarbamic acid,
- the isopropyl ester of 2-hydroxy-4-amino-5-methoxyphenylcarbamic acid,
- 3-(2-hydroxy-4-amino-5-methoxyphenyl)-1-(2-hydroxyethyl)urea,
- 3-(2-hydroxy-4-amino-5-methoxyphenyl)-1,1-di(2-hydroxyethyl)urea,
- the methyl ester of 2-hydroxy-4-methylamino-5-methoxyphenylcarbamic acid,
- the ethyl ester of 2-hydroxy-4-methylamino-5-methoxyphenylcarbamic acid,
- the 2-hydroxyethyl ester of 2-hydroxy-4-methylamino-5-methoxyphenylcarbamic acid,
- the isopropyl ester of 2-hydroxy-4-methylamino-5-methoxyphenylcarbamic acid,
- 1-(2-hydroxy-4-methylamino-5-methoxyphenyl)urea,
- 3-(2-hydroxy-4-methylamino-5-methoxyphenyl)-1-methylurea,
- 3-(2-hydroxy-4-methylamino-5-methoxyphenyl)-1-ethylurea,
- 3-(2-hydroxy-4-methylamino-5-methoxyphenyl)-1-(2-hydroxyethyl)urea,
- 3-(2-hydroxy-4-methylamino-5-methoxyphenyl)-1-isopropylurea,
- 3-(2-hydroxy-4-methylamino-5-methoxyphenyl)-1,1-dimethylurea,
- 3-(2-hydroxy-4-methylamino-5-methoxyphenyl)-1,1-diethylurea,
- 3-(2-hydroxy-4-methylamino-5-methoxyphenyl)-1,1-di(2-hydroxyethyl)urea,
- the methyl ester of 2-hydroxy-4-((2-hydroxyethyl)amino)-5-methoxyphenylcarbamic acid,
- the ethyl ester of 2-hydroxy-4-((2-hydroxyethyl)amino)-5-methoxyphenylcarbamic acid,
- the 2-hydroxyethyl ester of 2-hydroxy-4-((2-hydroxyethyl)amino)-5-methoxyphenylcarbamic acid,
- the isopropyl ester of 2-hydroxy-4-((2-hydroxyethyl)amino)-5-methoxyphenylcarbamic acid,
- 1-(2-hydroxy-4-((2-hydroxyethyl)amino)-5-methoxyphenyl)urea,
- 3-(2-hydroxy-4-((2-hydroxyethyl)amino)-5-methoxyphenyl)-1-methylurea,
- 3-(2-hydroxy-4-((2-hydroxyethyl)amino)-5-methoxyphenyl)-1-ethylurea,
- 3-(2-hydroxy-4-((2-hydroxyethyl)amino)-5-methoxyphenyl)-1-(2-hydroxyethyl)urea,
- 3-(2-hydroxy-4-((2-hydroxyethyl)amino)-5-methoxyphenyl)-1-isopropylurea,
- 3-(2-hydroxy-4-((2-hydroxyethyl)amino)-5-methoxyphenyl)-1,1-dimethylurea,
- 3-(2-hydroxy-4-((2-hydroxyethyl)amino)-5-methoxyphenyl)-1,1-diethylurea,
- 3-(2-hydroxy-4-((2-hydroxyethyl)amino)-5-methoxyphenyl)-1,1-di(2-hydroxyethyl)urea.

3. Composition according to Claim 1 or 2,
**characterized in that** the compounds of formula (I) are chosen from:
- the methyl ester of 2-hydroxy-4-aminophenyl-carbamic acid,
- the ethyl ester of 2-hydroxy-4-aminophenylcarbamic acid,
- the 2-hydroxyethyl ester of 2-hydroxy-4-aminophenylcarbamic acid,
- the isopropyl ester of 2-hydroxy-4-aminophenyl-carbamic acid,
- 1-(2-hydroxy-4-aminophenyl)urea,
- 3-(2-hydroxy-4-aminophenyl)-1-methylurea,
- 3-(2-hydroxy-4-aminophenyl)-1-ethylurea,
- 3-(2-hydroxy-4-aminophenyl)-1-(2-hydroxyethyl)urea,
- 3-(2-hydroxy-4-aminophenyl)-1-isopropylurea,
- 3-(2-hydroxy-4-aminophenyl)-1,1-dimethylurea,
- the methyl ester of 2-hydroxy-4-amino-5-chloro-phenylcarbamic acid,
- the ethyl ester of 2-hydroxy-4-amino-5-chloro-phenylcarbamic acid,
- the 2-hydroxyethyl ester of 2-hydroxy-4-amino-5-chlorophenylcarbamic acid,
- the isopropyl ester of 2-hydroxy-4-amino-5-chloro-phenylcarbamic acid,
- 1-(2-hydroxy-4-amino-5-chlorophenyl)urea,
- 3-(2-hydroxy-4-amino-5-chlorophenyl)-1-methylurea,
- 3-(2-hydroxy-4-amino-5-chlorophenyl)-1-ethylurea,
- 3-(2-hydroxy-4-amino-5-chlorophenyl)-1-(2-hydroxyethyl)urea,
- 3-(2-hydroxy-4-amino-5-chlorophenyl)-1-isopropyl-urea,
- 3-(2-hydroxy-4-amino-5-chlorophenyl)-1,1-dimethyl-urea,
- the methyl ester of 2-hydroxy-4-amino-5-methoxy-phenylcarbamic acid,
- the ethyl ester of 2-hydroxy-4-amino-5-methoxy-phenylcarbamic acid,
- the 2-hydroxyethyl ester of 2-hydroxy-4-amino-5-methoxyphenylcarbamic acid,
- the isopropyl ester of 2-hydroxy-4-amino-5-methoxyphenylcarbamic acid,
- 3-(2-hydroxy-4-amino-5-methoxyphenyl)-1-(2-hydroxyethyl)urea,
and their addition salts with an acid.

4. Composition according to any one of the preceding claims, **characterized in that** the compound or compounds of formula (I) and/or their addition salts with an acid preferably represent from 0.0005 to 12% by weight of the total weight of the dyeing composition.

5. Composition according to any one of the preceding claims, **characterized in that** the oxidation base or bases are chosen from para-phenylenediamines, bisphenylalkylenediamines, para-aminophenols, ortho-aminophenols and heterocyclic bases.

6. Composition according to Claim 5, **characterized in that** the para-phenylenediamines are chosen from para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis (β-hydroxyethyl)-para-phenylenediamine, 4-(N,N-bis (β-hydroxyethyl)amino)-2-methylaniline, 4-(N,N-bis (β-hydroxyethyl) amino)-2-chloroaniline, 2-(β-hydroxyethyl)-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-(β-hydroxyethyloxy)-para-phenylenediamine, 2-(β-acetylaminoethyloxy)-para-phenylenediamine, N-(β-methoxyethyl)-para-phenylenediamine and their addition salts with an acid.

7. Composition according to Claim 5, **characterized in that** the bisphenylalkylenediamines are chosen from N,N' -bis (β-hydroxyethyl) -N,N' -bis (4'-aminophenyl) -1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-diethyl-N,N'-bis(4'-amino-3'-methylphenyl)ethylenediamine, 1,8-bis(2,5-diaminophenoxy)-3,6-dioxaoctane and their addition salts with an acid.

8. Composition according to Claim 5, **characterized in that** the para-aminophenols are chosen from para-aminophenol, 4-amino-3-methylphenol, 4-amino-3-fluorophenol, 4-amino-3-(hydroxymethyl)phenol, 4-amino-2-methylphenol, 4-amino-2-(hydroxymethyl)phenol, 4-amino-2-(methoxymethyl)phenol, 4-amino-2-(aminomethyl)phenol, 4-amino-2-((β-hydroxyethyl)aminomethyl)phenol, 4-amino-2-fluorophenol and their addition salts with an acid.

9. Composition according to Claim 5, **characterized in that** the ortho-aminophenols are chosen from 2-aminophenol, 2-amino-5-methylphenol, 2-amino-6-methylphenol, 5-acetamido-2-aminophenol and their addition salts with an acid.

10. Composition according to Claim 5, **characterized in that** the heterocyclic bases are chosen from pyridine derivatives, pyrimidine derivatives and pyrazole derivatives.

11. Composition according to any one of the preceding claims, **characterized in that** the oxidation base or bases represent from 0.0005 to 12% by weight of the total weight of the dyeing composition.

12. Composition according to any one of the preceding claims, **characterized in that** it comprises one or more additional couplers chosen from meta-phenylenediamines, meta-aminophenols, meta-diphenols and heterocyclic couplers and/or at least one direct dye.

13. Composition according to Claim 12, **characterized in that** the additional coupler or couplers represent from 0.0001 to 10% by weight of the total weight of the dyeing composition.

14. Composition according to any one of the preceding claims, **characterized in that** the addition salts with an acid are chosen from hydrochlorides, hydrobromides, sulphates, citrates, succinates, tartrates, lactates and acetates.

15. Use of the compounds of formula (I) as defined in any one of Claims 1 to 3 as coupler for the oxidation dyeing of keratinous fibres and in particular of human keratinous fibres, such as the hair.

16. Method for dyeing keratinous fibres and in particular human keratinous fibres, such as the hair, **characterized in that** at least one dyeing composition as defined in any one of Claims 1 to 3 is applied to these fibres and **in that** the colour is developed at acidic, neutral or alkaline pH using an oxidizing agent which is added to the dyeing composition only at the time of use or which is present in an oxidizing composition applied simultaneously or sequentially.

17. Method according to Claim 16, **characterized in that** the oxidizing agent is chosen from hydrogen peroxide, urea hydrogen peroxide, alkali metal bromates, persalts and enzymes.

18. Method according to Claim 17, **characterized in that** the enzymes are chosen from peroxidases, laccases, tyrosinases and oxidoreductases.

19. Multicompartment device or multicompartment dyeing kit, a first compartment of which includes a dyeing composition as defined in any one of Claims 1 to 14 and a second compartment of which includes an oxidizing composition.

## Patentansprüche

1. Zusammensetzung zum oxidativen Färben von Keratinfasern,
**dadurch gekennzeichnet, dass** sie in einem zum Färben geeigneten Medium enthält:
- mindestens eine Oxidationsbase und
- mindestens einen Kuppler, der unter den Verbindungen der folgenden Formel (I) und ihren Additionssalzen mit einer Säure ausgewählt ist: 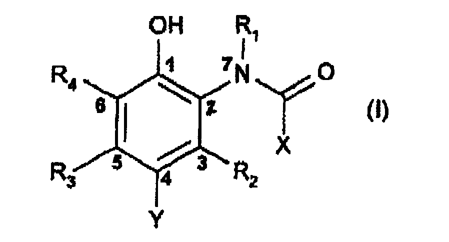
worin bedeuten:
- R₁ ein Wasserstoffatom;
- X Methoxy, Ethoxy, Propyloxy, Isopropyloxy, Butyloxy, Isobutyloxy, 2-Hydroxyethoxy, 2-Methoxyethoxy, Allyloxy, Amino, Methylamino, Ethylamino, Propylamino, Isopropylamino, Butylamino, Isobutylamino, 2-Hydroxyethylamino, 2-Methoxyethylamino, 3-Hydroxypropylamino, 2,3-Dihydroxypropylamino, Allylamino, Dimethylamino, Diethylamino, Di(2-hydroxyethyl)-amino, Pyrrolidin-1-yl, 3-Hydroxypyrrolidin-1-yl, 3, 4-Dihydroxypyrrolidin-1-yl;
- R₂ ein Wasserstoffatom oder die Methylgruppe;
- R₃ Amino, Methylamino, 2-Hydroxyethylamino, Diethylamino, Methansulfonylamino, Ethansulfonylamino oder Dimethylaminosulfonylamino;
- R₄ ein Wasserstoffatom, ein Chloratom oder ein Fluoratom; oder die Methylgruppe;
- Y ein Wasserstoffatom oder ein Chloratom; eine Methoxygruppe oder -OCH₂(CO)OCH₃.

2. Zusammensetzung nach Anspruch 1, **dadurch gekennzeichnet, dass** die Verbindungen in der Formel (I) ausgewählt sind unter:
- (2-Hydroxy-4-aminophenyl)-carbamidsäure-methylester,
- (2-Hydroxy-4-aminophenyl)-carbamidsäure-ethylester,
- (2-Hydroxy-4-aminophenyl)-carbamidsäure-2-hydroxyethylester,
- (2-Hydroxy-4-aminophenyl)-carbamidsäure-isopropylester,
- 1-(2-Hydroxy-4-aminophenyl)-harnstoff,
- 3-(2-Hydroxy-4-aminophenyl)-1-methyl-harnstoff,
- 3-(2-Hydroxy-4-aminophenyl)-1-ethyl-harnstoff,
- 3-(2-Hydroxy-4-aminophenyl)-1-(2-hydroxyethyl)-harnstoff,
- 3-(2-Hydroxy-4-aminophenyl)-1-isopropyl-harnstoff,
- 3-(2-Hydroxy-4-aminophenyl)-1, 1 -dimethyl-harnstoff,
- 3-(2-Hydroxy-4-aminophenyl)-1,1-diethyl-harnstoff,
- 3-(2-Hydroxy-4-aminophenyl)- 1, 1 -di-(2-hydroxyethyl)-harnstoff,
- Pyrrolidin-1-carbonsäure-(4-amino-2-hydroxyphenyl)-amid,
- 3-Hydroxypyrrolidin-1-carbonsäure-(4-amino-2-hydroxyphenyl)-amid,
- (2-Hydroxy-4-methylaminophenyl)-carbamidsäure-methylester,
- (2-Hydroxy-4-methylaminophenyl)-carbamidsäure-ethylester,
- (2-Hydroxy-4-methylaminophenyl)-carbamidsäure-2-hydroxy-ethylester,
- (2-Hydroxy-4-methylaminophenyl)-carbamidsäure-isopropylester,
- 1-(2-Hydroxy-4-methylaminophenyl)-harnstoff,
- 3-(2-Hydroxy-4-methylaminophenyl)-1-methyl-harnstoff,
- 3-(2-Hydroxy-4-methylaminophenyl)-1-ethyl-harnstoff,
- 3-(2-Hydroxy-4-methylaminophenyl)-1-(2-hydroxyethyl)-harnstoff,
- 3-(2-Hydroxy-4-methylaminophenyl)-1-isopropyl-harnstoff,
- 3-(2-Hydroxy-4-methylaminophenyl)-1, 1-dimethyl-harnstoff,
- 3-(2-Hydroxy-4-methylaminophenyl)- 1, 1 -diethyl-harnstoff,
- 3-(2-Hydroxy-4-methylaminophenyl)-1, 1-di(2-hydroxyethyl)-harnstoff,
- (2-Hydroxy-4-(2-hydroxyethyl)amino-phenyl)-carbamidsäure-methylester,
- (2-Hydroxy-4-(2-hydroxyethyl)amino-phenyl)-carbamidsäure-ethylester,
- (2-Hydroxy-4-(2-hydroxyethyl)amino-phenyl)-carbamidsäure-2-hydroxyethylester,
- (2-Hydroxy-4-(2-hydroxyethyl)amino-phenyl)-carbamidsäure-isopropylester,
- 1-(2-Hydroxy-4-(2-hydroxyethyl)amino-phenyl)-harnstoff,
- 3-(2-Hydroxy-4-(2-hydroxyethyl)amino-phenyl)-1-methylharnstoff,
- 3-(2-Hydroxy-4-(2-hydroxyethyl)amino-phenyl)-1-ethyl-harnstoff,
- 3-(2-Hydroxy-4-(2-hydroxyethyl)amino-phenyl)-1-(2-hydroxyethyl)-harnstoff,
- 3-(2-Hydroxy-4-(2-hydroxyethyl)amino-phenyl)-1-isopropyl-harnstoff,
- 3-(2-Hydroxy-4-(2-hydroxyethyl)amino-phenyl)-1,1-dimethyl-harnstoff,
- 3-(2-Hydroxy-4-(2-hydroxyethyl)amino-phenyl)-1,1-diethyl-harnstoff,
- 3-(2-Hydroxy-4-(2-hydroxyethyl)amino-phenyl)-1,1-di(2-hydroxyethyl)-harnstoff,
- (2-Hydroxy-4-amino-5-chlorphenyl)-carbamidsäure-methylester,
- (2-Hydroxy-4-amino-5-chlorphenyl)-carbamidsäure-ethylester,
- (2-Hydroxy-4-amino-5-chlorphenyl)-carbamidsäure-2-hydroxyethylester,
- (2-Hydroxy-4-amino-5-chlorphenyl)-carbamidsäure-iso-propylester,
- 1-(2-Hydroxy-4-amino-5-chlorphenyl)-harnstoff,
- 3-(2-Hydroxy-4-amino-5-chlorphenyl)-1-methyl-harnstoff,
- 3-(2-Hydroxy-4-amino-5-chlorphenyl)-1-ethyl-harnstoff,
- 3-(2-Hydroxy-4-amino-5-chlorphenyl)-1-(2-hydroxyethyl)-harnstoff,
- 3-(2-Hydroxy-4-amino-5-chlorphenyl)-1-isopropyl-harnstoff,
- 3-(2-Hydroxy-4-amino-5-chlorphenyl)-1,1-dimethyl-harnstoff,
- 3-(2-Hydroxy-4-amino-5-chlorphenyl)-1, 1-diethyl-harnstoff,
- 3-(2-Hydroxy-4-amino-5-chlorphenyl)-1,1-di(2-hydroxyethyl)-harnstoff,
- (2-Hydroxy-4-methylamino-5-chlorphenyl)-carbamidsäure-methylester,
- (2-Hydroxy-4-methylamino-5-chlorphenyl)-carbamidsäure-ethylester,
- (2-Hydroxy-4-methylamino-5-chlorphenyl)-carbamidsäure-2-hydroxyethylester,
- (2-Hydroxy-4-methylamino-5-chlorphenyl)-carbamidsäure-isopropylester,
- 1-(2-Hydroxy-4-methylamino-5-chlorphenyl)-harnstoff,
- 3-(2-Hydroxy-4-methylamino-5-chlorphenyl)-1-methylharnstoff,
- 3-(2-Hydroxy-4-methylamino-5-chlorphenyl)-1-ethyl-harnstoff,
- 3-(2-Hydroxy-4-methylamino-5-chlorphenyl)-1-(2-hydroxyethyl)-harnstoff,
- 3-(2-Hydroxy-4-methylamino-5-chlorphenyl)-1-isopropyl-harnstoff,
- 3-(2-Hydroxy-4-methylamino-5-chlorphenyl)-1, 1-dimethyl-harnstoff,
- 3-(2-Hydroxy-4-methylamino-5-chlorphenyl)-1, 1 -diethyl-harnstoff,
- 3-(2-Hydroxy-4-methylamino-5-chlorphenyl)-1,1-di(2-hydroxyethyl)-harnstoff,
- (2-Hydroxy-4-(2-hydroxyethyl)amino-5-chlorphenyl)-carbamidsäure-methylester,
- (2-Hydroxy-4-(2-hydroxyethyl)amino-5-chlorphenyl)-carbamidsäure-ethylester,
- (2-Hydroxy-4-(2-hydroxyethyl)amino-5-chlorphenyl)-carbamidsäure-2-hydroxyethylester,
- (2-Hydroxy-4-(2-hydroxyethyl)amino-5-chlorphenyl)-carbamidsäure-isopropylester,
- 1-(2-Hydroxy-4-(2-hydroxyethyl)amino-5-chlorphenyl)-harnstoff,
- 3-(2-Hydroxy-4-(2-hydroxyethyl)amino-5-chlorphenyl)-1-methylharnstoff,
- 3-(2-Hydroxy-4-(2-hydroxyethyl)amino-5-chlorphenyl)-1-ethylharnstoff,
- 3-(2-Hydroxy-4-(2-hydroxyethyl)amino-5-chlorphenyl)-1-(2-hydroxyethyl)-harnstoff,
- 3-(2-Hydroxy-4-(2-hydroxyethyl)amino-5-chlorphenyl)-1-isopropyl)-harnstoff,
- 3-(2-Hydroxy-4-(2-hydroxyethyl)amino-5-chlorphenyl)-1,1-dimethyl-harnstoff,
- 3-(2-Hydroxy-4-(2-hydroxyethyl)amino-5-chlorphenyl)-1,1-diethyl-harnstoff,
- 3-(2-Hydroxy-4-(2-hydroxyethyl)amino-5-chlorphenyl)-1,1-di(2-hydroxyethyl)-harnstoff,
- (2-Hydroxy-4-amino-5-methoxyphenyl)-carbamidsäure-methylester,
- (2-Hydroxy-4-amino-5-methoxyphenyl)-carbamidsäure-ethylester,
- (2-Hydroxy-4-amino-5-methoxyphenyl)-carbamidsäure-2-hydroxyethylester,
- (2-Hydroxy-4-amino-5-methoxyphenyl)-carbamidsäure-isopropylester,
- 3-(2-Hydroxy-4-amino-5-methoxyphenyl)-1-(2-hydroxyethyl)-harnstoff,
- 3-(2-Hydroxy-4-amino-5-methoxyphenyl)-1,1-di(2-hydroxyethyl)-harnstoff,
- (2-Hydroxy-4-methylamino-5-methoxyphenyl)-carbamidsäure-methylester,
- (2-Hydroxy-4-methylamino-5-methoxyphenyl)-carbamidsäure-ethylester,
- (2-Hydroxy-4-methylamino-5-methoxyphenyl)-carbamidsäure-2-hydroxyethylester,
- (2-Hydroxy-4-methylamino-5-methoxyphenyl)-carbamidsäure-isopropylester,
- 1-(2-Hydroxy-4-methylamino-5-methoxyphenyl)-harnstoff,
- 3-(2-Hydroxy-4-methylamino-5-methoxyphenyl)-1-methylharnstoff,
- 3-(2-Hydroxy-4-methylamino-5-methoxyphenyl)-1-ethyl-harnstoff,
- 3-(2-Hydroxy-4-methylamino-5-methoxyphenyl)-1-(2-hydroxyethyl)-harnstoff,
- 3-(2-Hydroxy-4-methylamino-5-methoxyphenyl)-1-isopropyl-harnstoff,
- 3-(2-Hydroxy-4-methylamino-5-methoxyphenyl)-1,1-dimethyl-harnstoff,
- 3-(2-Hydro-4-methylamino-5-methoxyphenyl)-1,1-diethyl-harnstoff,
- 3-(2-Hydroxy-4-methylamino-5-methoxyphenyl)-1,1-di(2-hydroxyethyl)-harnstoff,
- (2-Hydroxy-4-(2-hydroxyethyl)amino-5-methoxyphenyl)-carbamidsäure-methylester,
- (2-Hydroxy-4-(2-hydroxyethyl)amino-5-methoxyphenyl)-carbamidsäure-ethylester,
- (2-Hydroxy-4-(2-hydroxyethyl)amino-5-methoxyphenyl)-carbamidsäure-2-hydroxyethylester,
- (2-Hydroxy-4-(2-hydroxyethyl)amino-5-methoxyphenyl)-carbamidsäure-isopropylester,
- 1-(2-Hydroxy-4-(2-hydroxyethyl)amino-5-methoxyphenyl)-harnstoff,
- 3-(2-Hydroxy-4-(2-hydroxyethyl)amino-5-methoxyphenyl)-1-methyl-harnstoff,
- 3-(2-Hydroxy-4-(2-hydroxyethyl)amino-5-methoxyphenyl)-1-ethyl-harnstoff,
- 3-(2-Hydroxy-4-(2-hydroxyethyl)amino-5-methoxyphenyl)-1-(2-hydroxyethyl)-harnstoff,
- 3-(2-Hydroxy-4-(2-hydroxyethyl)amino-5-methoxyphenyl)-1-isopropyl-harnstoff,
- 3-(2-Hydroxy-4-(2-hydroxyethyl)amino-5-methoxyphenyl)-1,1-dimethyl-harnstoff,
- 3-(2-Hydroxy-4-(2-hydroxyethyl)amino-5-methoxyphenyl)- 1, 1 - diethyl-harnstoff,
- 3-(2-Hydroxy-4-(2-hydroxyethyl)amino-5-methoxyphenyl)-1,1-di(2-hydroxyethyl)-harnstoff.

3. Zusammensetzung nach Anspruch 1 oder 2, **dadurch gekennzeichnet, dass** die Verbindungen der Formel (I) ausgewählt sind unter:
- (2-Hydroxy-4-aminophenyl)-carbamidsäure-methylester,
- (2-Hydroxy-4-aminophenyl)-carbamidsäure-ethylester,
- (2-Hydroxy-4-aminophenyl)-carbamidsäure-2-hydroxyethylester,
- (2-Hydroxy-4-aminophenyl)-carbamidsäure-isopropylester,
- 1-(2-Hydroxy-4-aminophpenyl)-harnstoff,
- 3-(2-Hydroxy-4-aminophenyl)-1-methyl-harnstoff,
- 3-(2-Hydroxy-4-aminophenyl)-1-ethyl-harnstoff,
- 3-(2-Hydroxy-4-aminophenyl)-1-(2-hydroxyethyl)-harnstoff,
- 3-(2-Hydroxy-4-aminophenyl)-1-isopropyl-harnstoff,
- 3-(2-Hydroxy-4-aminophenyl)-1, 1-dimethyl-harnstoff,
- (2-Hydroxy-4-amino-5-chlorphenyl)-carbamidsäure-methylester,
- (2-Hydroxy-4-amino-5-chlorphenyl)-carbamidsäure-ethylester,
- (2-Hydroxy-4-amino-5-chlorphenyl)-carbamidsäure-2-hydroxyethylester,
- (2-Hydroxy-4-amino-5-chlorphenyl)-carbamidsäure-isopropylester,
- 1-(2-Hydroxy-4-amino-5-chlorphenyl)-harnstoff,
- 3-(2-Hydroxy-4-amino-5-chlorphenyl)-1-methyl-harnstoff,
- 3-(2-Hydroxy-4-amino-5-chlorphenyl)-1-ethyl-harnstoff,
- 3-(2-Hydroxy-4-amino-5-chlorphenyl)-1-(2-hydroxyethyl)-harnstoff,
- 3-(2-Hydroxy-4-amino-5-chlorphenyl)-1-isopropyl-harnstoff,
- 3-(2-Hydroxy-4-amino-5-chlorphenyl)-1,1-dimethyl-harnstoff,
- (2-Hydroxy-4-amino-5-methoxyphenyl)-carbamidsäure-methylester,
- (2-Hydroxy-4-amino-5-methoxyphenyl)-carbamidsäure-ethylester,
- (2-Hydroxy-4-amino-5-methoxyphenyl)-carbamidsäure-2-hydroxy-ethylester,
- (2-Hydroxy-4-amino-5-methoxyphenyl)-carbamidsäure-isopropylester,
- 3-(2-Hydroxy-4-amino-5-methoxyphenyl)-1-(2-hydroxyethyl)-harnstoff
und deren Additionssalzen mit einer Säure.

4. Zusammensetzung nach einem der vorhergehenden Ansprüche, **dadurch gekennzeichnet, dass** die Verbindung(en) der Formel (I) und/oder das oder die Additionssalze dieser Verbindung(en) mit einer Säure vorzugsweise 0,0005 bis 12 Gew.-% des Gesamtgewichts der Farbmittelzusammensetzung ausmachen.

5. Zusammensetzung nach einem der vorhergehenden Ansprüche, **dadurch gekennzeichnet, dass** die Oxidationsbase(n) unter den *p*-Phenylendiaminen, Bisphenylalkylendiaminen, *p*-Aminophenolen, o-Aminophenolen und heterocyclischen Basen ausgewählt sind.

6. Zusammensetzung nach Anspruch 5, **dadurch gekennzeichnet, dass** die p-Phenylendiamine unter p-Phenylendiamin, *p-*Toluylendiamin, 2-Chlor-*p*-phenylendiamin, 2,3₋Dimethyl-*p-*phenylendiamin, 2,6-Dimethyl-*p*-phenylendiamin, 2,6-Diethyl*p*-phenylendiamin, 2,5-Dimethyl-*p*-phenylendiamin, N,N-Dimethyl-*p*-phenylendiamin, N,N-Diethyl-*p*-phenylendiamin, N,N-Dipropyl-*p*-phenylendiamin, 4-Amino-N,N-diethyl-3-methylanilin, N,N-Bis(β-hydroxyethyl)-*p*-phenylendiamin, 4-N,N-Bis(β-hydroxyethyl)amino-2-methylanilin, 4-N,N-Bis(β-hydroxyethyl)amino-2-chloranilin, 2-β-Hydroxyethyl-*p*-phenylendiamin, 2-Fluor-*p*-phenylendiamin, 2-Isopropyl-p-phenylendiamin, N-(β-Hydro propyl)-p-phenylendiamin, 2-Hydroxymethyl-*p-*phenylendiamin, N,N-Dimethyl-3-methyl-p-phenylendiamin, N,N-(Ethyl,β-hydroxyethyl)-*p*-phenylendiamin, N-(β,γ-Dihydroxypropyl)-p-phenylendiamin, N-(4'-Aminophenyl)-*p*-phenylendiamin, N-Phenyl-*p*-phenylendiamin, 2-β-Hydroxyethyloxy-*p-*phenylendiamin, 2-β-Acetylaminoethyloxy-*p*-phenylendiamin, N-(β-Methoxyethyl)-*p*-phenylendiamin und ihren Additionssalzen mit einer Säure ausgewählt sind.

7. Zusammensetzung nach Anspruch 5, **dadurch gekennzeichnet, dass** die Bisphenylalkylendiamine unter N,N'-Bis(β-hydroxyethyl)-N,N'-bis(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methylaminophenyl)-tetramethylendiamin, N,N'-Bis(ethyl)-N,N'-bis(4'-amino, 3'-methylphenyl)-ethylendiamin, 1,8-Bis(2,5-diaminophenoxy)-3,5-dioxaoctan und ihren Additionssalzen mit einer Säure ausgewählt sind.

8. Zusammensetzung nach Anspruch 5, **dadurch gekennzeichnet, dass** die *p*-Aminophenole unter p-Aminophenol, 4-Amino-3-methylphenol, 4-Amino-3-fluorphenol, 4-Amino-3-hydroxy-methylphenol, 4-Amino-2-methylphenol, 4-Amino-2-hydroxy-methylphenol, 4-Amino-2-methoxymethylphenol, 4-Amino-2-aminomethylphenol, 4-Amino-2-(β-hydroxyethyl-aminomethyl)-phenol, 4-Amino-2-fluorphenol und ihren Additionssalzen mit einer Säure ausgewählt sind.

9. Zusammensetzung nach Anspruch 5, **dadurch gekennzeichnet, dass** die o-Aminophenole unter 2-Aminophenol, 2-Amino-5-methylphenol, 2-Amino-6-methylphenol, 5-Acetamido-2-aminophenol und ihren Additionssalzen mit einer Säure ausgewählt sind.

10. Zusammensetzung nach Anspruch 5, **dadurch gekennzeichnet, dass** die heterocyclischen Basen unter den Pyridinderivaten, Pyrimidinderivaten und Pyrazolderivaten ausgewählt sind.

11. Zusammensetzung nach einem der vorhergehenden Ansprüche, **dadurch gekennzeichnet, dass** die Oxidationsbase oder die Oxidationsbasen 0,0005 bis 12 Gew.-% des Gesamtgewichts der Farbmittelzusammensetzung ausmachen.

12. Zusammensetzung nach einem der vorhergehenden Ansprüche, **dadurch gekennzeichnet, dass** sie einen oder mehrere ergänzende Kuppler, die unter den *m*-Phenylendiaminen, *m*-Aminophenolen, *m*-Dihydroxybenzolen und heterocyclischen Kupplern ausgewählt sind, und/oder mindestens einen Direktfarbstoff enthält.

13. Zusammensetzung nach Anspruch 12, **dadurch gekennzeichnet, dass** der oder die ergänzenden Kuppler 0,0001 bis 10 Gew.-% des Gesamtgewichts der Farbmittelzusammensetzung ausmachen.

14. Zusammensetzung nach einem der vorhergehenden Ansprüche, **dadurch gekennzeichnet, dass** die Additionssalze mit einer Säure unter den Hydrochloriden, Hydrobromiden, Sulfaten, Citraten, Succinaten, Tartraten, Lactaten und Acetaten ausgewählt sind.

15. Verwendung von Verbindungen der Formel (I) und einem der Ansprüche 1 bis 3 als Kuppler zum oxidativen Färben von Keratinfasern und insbesondere menschlichen Keratinfasern, wie Haaren.

16. Verfahren zum Färben von Keratinfasern und insbesondere menschlichen Keratinfasern wie Haaren, **dadurch gekennzeichnet, dass** auf die Fasern mindestens eine Farbmittelzusammensetzung nach einem der Ansprüche 1 bis 3 aufgetragen wird und die Farbe bei einem sauren, neutralen oder alkalischen pH-Wert mit einem Oxidationsmittel gebildet wird, das bei der Anwendung zu der Farbmittelzusammensetzung gegeben wird oder das in einer oxidierenden Zusammensetzung enthalten ist, die gleichzeitig oder getrennt davon anschließend aufgetragen wird.

17. Verfahren nach Anspruch 16, **dadurch gekennzeichnet, dass** das Oxidationsmittel unter Wasserstoffperoxid, Harnstoffperoxid, Alkalimetallbromaten, Persalzen und Enzymen ausgewählt ist

18. Verfahren nach Anspruch 17, **dadurch gekennzeichnet, dass** die Enzyme unter den Peroxidasen, Laccasen, Tyrosinasen und Oxidoreductasen ausgewählt sind.

19. Vorrichtung mit mehreren Abteilungen oder "Kit" zum Färben mit mehreren Abteilungen, wobei eine erste Abteilung eine Farbmittelzusammensetzung nach einem der Ansprüche 1 bis 14 enthält und eine zweite Abteilung eine oxidierende Zusammensetzung enthält.
